# FASCICULE DE BREVET EUROPEEN

(11) **EP 2 389 856 B1**
(45) Date de publication et mention de la délivrance du brevet: **16.11.2016**
(21) Numéro de dépôt: 11290221.8
(22) Date de dépôt: 11.05.2011
(51) Int. Cl.: A61B 3/02, G01C 13/00, G01S 15/88, A61B 3/032

(54) **Dispositif de mesure d'une distance caractéristique de lecture**
Vorrichtung zur Messung eines charakteristischen Leseabstands
Device for measuring a typical reading distance

(30) Priorité: 25.05.2010 FR 1002200
(43) Date de publication de la demande: 30.11.2011
(73) Titulaire: Essilor International, 94220 Charenton Le Pont (FR)
(72) Inventeur: Haddadi, Ahmed , Essilor International, 94220 Charenton-le-Pont (FR)
(74) Mandataire: Chauvin, Vincent

(56) Documents cités:
- EP-A1- 1 426 919
- WO-A1-2008/032982
- WO-A1-2008/064379
- DE-C1- 3 808 260
- JP-A- 2000 325 309
- JP-A- 2009 160 197
- US-A- 5 596 379

## Description

### DOMAINE TECHNIQUE AUQUEL SE RAPPORTE L'INVENTION

La présente invention concerne un dispositif de mesure d'une distance caractéristique de lecture d'un individu.

### ARRIÈRE-PLAN TECHNOLOGIQUE

La détermination de la distance caractéristique de lecture d'un individu est très importante pour la réalisation de lentilles ophtalmiques procurant à leur porteur une correction optique personnalisée et particulièrement adaptée à la vision de près de l'individu.

On connait du document JP2000-325309 un dispositif pour déterminer un telle distance caractéristique et qui comporte, d'une part, un support de lecture réalisé sous la forme d'une tablette placée à une distance adaptée à la lecture d'un texte porté par cette tablette et, d'autre part, des moyens de mesure de la distance de la tête par rapport à cette tablette. Ces moyens de mesure de distance procèdent par ultrasons : un émetteur et un récepteur d'ultrasons sont prévus sur la tablette. Le récepteur détecte les ultrasons émis par l'émetteur et réfléchis sur la tête de l'individu. Cette méthode présente l'inconvénient d'être peu précise.

On connaît également du document US5596379 un dispositif de mesure de la distance de lecture d'un individu comportant un support de mesure et des moyens de mesure par ultrasons de la distance entre l'individu et le dispositif. Un faisceau laser émis par le dispositif permet en outre à l'opérateur de visualiser si l'individu est bien disposé en face du dispositif de mesure de manière à assurer que la distance mesurée est bien celle entre l'individu et le dispositif. La mesure réalisée par ce dispositif présente également l'inconvénient d'être peu précise.

### OBJET DE L'INVENTION

Un but de la présente invention est de proposer un dispositif de mesure d'une distance caractéristique de lecture permettant une mesure rapide, précise et facile à mettre en oeuvre, avec aussi peu de contrainte que possible sur la posture (notamment éloignement et inclinaison autour de l'horizontale) de l'individu par rapport à un support de lecture. On souhaite en particulier s'affranchir de la nécessité de rapporter sur la monture ou sur la tête de l'individu un accessoire de repérage dans l'espace.

A cet effet, on propose selon l'invention un dispositif de mesure d'une distance caractéristique de lecture d'un individu tel que décrit dans la revendication 1.

La mesure est effectuée selon un principe de mesure de distance par ultrasons : l'organe récepteur détecte des signaux ultrasons émis par l'émetteur et réfléchis sur la monture de lunettes disposée sur la tête de l'individu.

En pratique, il est particulièrement avantageux de réaliser cette mesure par ultrasons, car la différence de réflectivité entre la peau de l'individu et la monture de lunettes disposée sur la tête de l'individu est importante et permet de distinguer facilement le signal réfléchi par cette monture.

La distance caractéristique de lecture est ici déduite de la distance entre la monture de lunettes de l'individu et le dispositif de mesure.

Une première caractéristique avantageuse et non limitative du dispositif est énoncée dans la revendication 2.

Afin de réaliser la mesure aisément, il est avantageux d'utiliser un organe émetteur d'ultrasons émettant dans un angle solide qui couvrira facilement l'ensemble de la tête de l'individu ou du moins la monture équipant la tête de l'individu, c'est-à-dire émettant les ultrasons dans un cône de mesure d'angle au sommet supérieur ou égal à 15 degrés.

Afin de réaliser une mesure précise, il est avantageux d'utiliser un organe de mesure principal directif, c'est-à-dire émettant ou recevant les ultrasons exclusivement dans un cône de mesure d'angle au sommet inférieur ou égal à 45 degrés.

Il est alors utile de vérifier que la tête de l'individu, et plus précisément la monture de lunettes équipant la tête de l'individu se trouve bien dans ce cône de mesure.

Le dispositif de mesure comporte à cet effet des moyens pour vérifier que la monture de lunettes est au moins partiellement située dans le cône de mesure dudit organe de mesure principal.

Selon un premier mode de réalisation du dispositif selon l'invention, le dispositif de mesure est tel que décrit dans l'une des revendications 3 et 4.

Le support de mesure peut alors avantageusement être fixé à différents types de support de lecture adapté à l'individu, par exemple, sur une bande-dessinée pour les enfants, sur un livre ou un magazine, ou sur tout type d'écran d'affichage.

Selon un deuxième mode de réalisation du dispositif selon l'invention, le dispositif de mesure est tel que décrit dans l'une des revendications 5 et 6.

Avantageusement, les moyens de raccordement décrits dans la revendication 6 sont tels que, après raccordement, le plan de la partie d'affichage soit oblique par rapport au plan sur lequel repose l'embase.

Le support de lecture peut alors être un appareil portable propre à l'individu, du type téléphone portable ou tablette multimédia.

Quel que soit le mode de réalisation du dispositif de mesure, celui-ci peut avantageusement présenter les caractéristiques de l'une des revendications 7 à 10.

Les moyens pour vérifier visuellement que la tête de l'individu est au moins partiellement située dans le cône de mesure comportent par exemple au moins une cible et un marquage solidaires dudit organe de mesure principal, le marquage et la cible étant décalés l'un de l'autre suivant la direction de l'axe de mesure de l'organe de mesure principal. La tête de l'individu est au moins partiellement située dans le cône de mesure de l'organe de mesure lorsqu'il constate visuellement le centrage de ladite cible sur ledit marquage.

En variante, ces moyens de vérification comportent par exemple au moins une cible solidaire dudit organe de mesure principal et disposée au fond d'une cavité de telle sorte que la cible ne soit visible par l'individu que dans une plage angulaire prédéterminée d'orientation de l'organe de mesure par rapport à la tête de l'individu.

Une autre caractéristique avantageuse et non limitative du dispositif est énoncée dans la revendication 11.

L'unité de traitement électronique est alors adaptée à déduire la distance caractéristique de lecture recherchée qui correspond à la distance entre la monture de lunettes de l'individu et le support de lecture de la distance mesurée entre le support de mesure et la monture de lunettes de l'individu et de la position relative connue du support de mesure et du support de lecture.

Alternativement, le dispositif de mesure est tel que décrit dans la revendication 12.

L'unité de traitement électronique est alors adaptée à déduire de la distance mesurée entre le support de mesure et la monture de lunettes de l'individu et de la position relative déterminée du support de mesure par rapport au support de lecture la distance caractéristique de lecture recherchée qui correspond à la distance entre la monture de lunettes de l'individu et le support de lecture.

En particulier, les moyens pour déterminer la position relative du support de lecture sont tels que décrits dans la revendication 13.

Une autre caractéristique avantageuse et non limitative du dispositif de mesure est énoncée dans la revendication 14.

Le confort de l'individu est ainsi augmenté et la précision de la mesure est améliorée : l'individu peut se placer plus facilement dans une position de lecture naturelle pour lui.

Une autre caractéristique avantageuse et non limitative du dispositif de mesure est énoncée dans la revendication 15.

La cavité conique constitue un guide d'onde pour l'émission et la réception des ultrasons et permet d'amplifier le signal reçu. La précision de la mesure est ainsi améliorée.

### DESCRIPTION DETAILLEE D'UN EXEMPLE DE REALISATION

La description qui va suivre, en regard des dessins annexés, donnée à titre d'exemple non limitatif, fera bien comprendre en quoi consiste l'invention et comment elle peut être réalisée.

Sur les dessins annexés :
- la figure 1 est une vue en perspective avant d'un premier mode de réalisation du dispositif de mesure selon l'invention,
- la figure 2 est une vue en perspective arrière du dispositif de mesure de la figure 1,
- la figure 3 est une vue en perspective arrière d'une première variante du dispositif de mesure de la figure 1,
- la figure 4 est une vue de dessus d'une deuxième variante du dispositif de mesure de la figure 1,
- la figure 5 est une vue de face du dispositif de mesure de la figure 4,
- la figure 6 est une vue de profil d'une troisième variante du dispositif de mesure de la figure 1,
- la figure 7 est une vue schématique d'un individu utilisant le dispositif de l'une des figures 1 à 5,
- la figure 8 est une vue en perspective avant d'une première variante d'un deuxième mode de réalisation du dispositif de mesure selon l'invention,
- la figure 9 est une vue schématique d'un individu utilisant le dispositif de la figure 8,
- la figure 10 est une vue analogue à la figure 9, illustrant une seconde variante du deuxième mode de réalisation du dispositif de mesure selon l'invention,
- la figure 11 est une vue schématique en élévation de profil d'un individu utilisant le dispositif de la figure 1.

Dans la description qui suit, l'opticien détermine une distance de lecture caractéristique d'un individu.

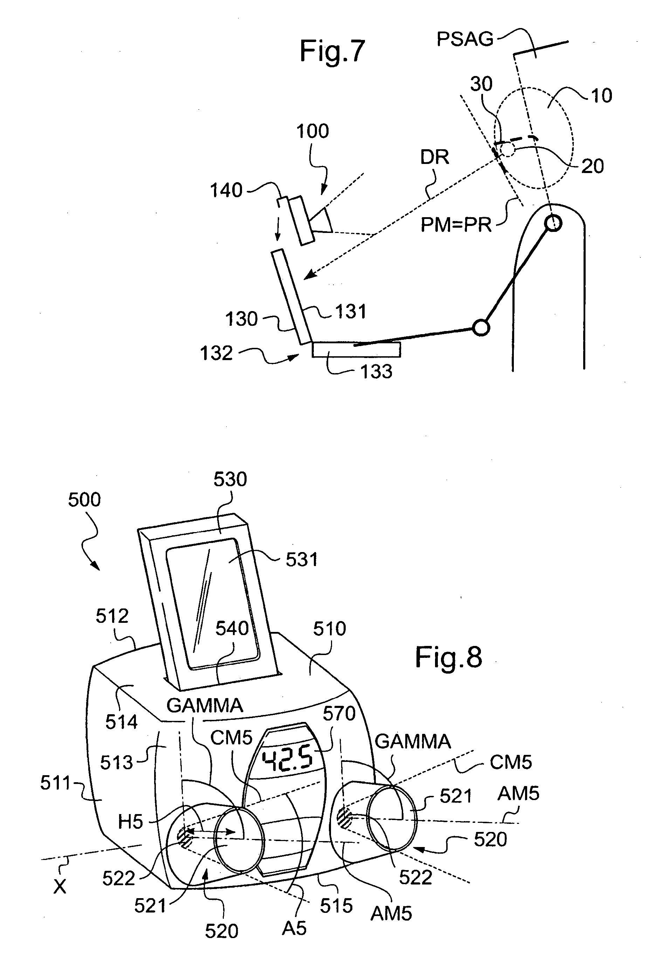

Comme le montrent les figures 7, 9, 10 et 11, la tête 10 de l'individu est équipée d'une monture 30 de lunettes munie de lentilles ophtalmiques de correction visuelle.

L'individu est dans une configuration assise ou debout.

On définit ici la direction verticale comme la direction donnée par un fil à plomb en un lieu déterminé. Une direction horizontale est perpendiculaire à cette direction verticale.

Un plan sagittal PSAG de la tête 10 de l'individu est un plan médian de la tête de l'individu passant par le milieu des deux centres de rotation des yeux 20 de l'individu et perpendiculaire à la droite reliant ces deux centres de rotation des yeux 20. Le plan sagittal est sensiblement vertical lorsque l'individu est assis ou debout en posture naturelle.

On définit une direction de regard DR comme la droite bissectrice de l'angle entre les droites reliant chaque oeil de l'individu au point fixé du regard par l'individu.

On définit également un plan de regard PR perpendiculaire à la direction de regard DR. En première approximation, le plan de regard PR est confondu avec le plan moyen PM de la monture 30.

Le plan moyen PM de la monture 30 de lunettes est ici confondu avec les plans moyens des lentilles ophtalmiques correspondantes.

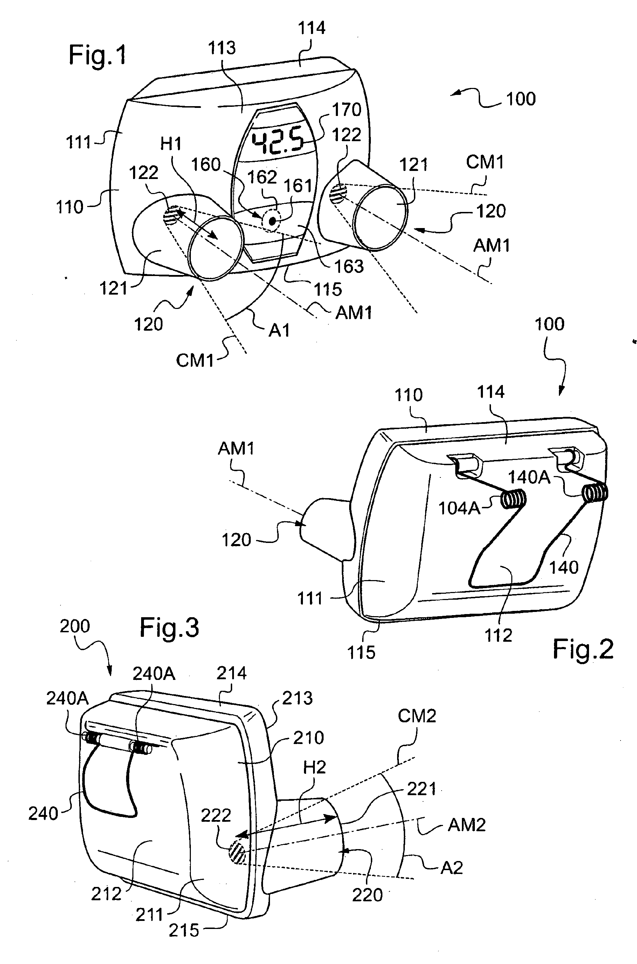

On a représenté sur les figures 1 à 6 différentes variantes d'un premier mode de réalisation du dispositif de mesure 100; 200; 300; 400 selon l'invention et, sur la figure 8, un deuxième mode de réalisation du dispositif 500 selon l'invention.

Ce dispositif de mesure 100; 200; 300; 400; 500; 600 comporte ici un support de mesure 110, 210; 310; 410; 510 portant au moins un organe émetteur et au moins un organe récepteur d'ultrasons.

Le support de mesure 110, 210; 310; 410; 510; 610 comporte un boîtier 111; 211; 311; 411; 511; 611 de forme globalement parallélépipédique avec une face supérieure 114; 214; 314; 414; 514 et une face inférieure 115; 215; 315; 415; 515 opposées.

Le boîtier 111; 211; 311; 411; 511; 611 porte deux organes émetteurs-récepteurs 120; 220; 320; 420; 520; 620.

Ces organes émetteurs-récepteurs sont accessibles en face avant 113; 213; 313; 413; 513 du boîtier.

Cette face avant est orientée vers la tête de l'individu.

Chaque organe émetteur-récepteur 120; 220; 320; 420; 520; 620 possède un axe de mesure AM1; AM2; AM3; AM4; AM5 et est adapté à émettre ou à recevoir les ultrasons se propageant dans un cône de mesure CM1; CM2; CM3; CM4; CM5 centré sur son axe de mesure.

Chaque organe émetteur-récepteur 120; 220; 320; 420; 520; 620 comporte un élément émetteur-récepteur 122; 222; 322; 422; 522 qui est apte à émettre et à recevoir des ultrasons. Cet élément émetteur-récepteur est placé au fond d'une cavité 121; 221; 321; 421; 521 ménagée dans le boîtier. Cette cavité 121; 221; 321; 421; 521 présente ici une paroi intérieure de forme conique 121; 221; 321; 421; 521.

On entend ici par axe de mesure un axe selon lequel l'émission ou la réception par l'organe de mesure est privilégiée.

Dans le cas d'un organe émetteur-récepteur, l'axe de mesure correspond à l'axe selon lequel un signal d'intensité maximale est détecté pour un signal émis d'intensité déterminée.

Pour un organe récepteur, cet axe de mesure correspond à l'axe passant par l'élément récepteur selon lequel l'intensité du signal ultrasons reçu est maximale lorsque cet axe pointe vers une source d'ultrasons. Pour un organe émetteur, cet axe de mesure correspond à l'axe passant par l'élément émetteur selon lequel l'intensité du signal ultrasons émis est maximale lorsque cet axe pointe vers un élément récepteur d'ultrasons.

En pratique, l'axe de mesure correspond à un axe de symétrie de la cavité 121; 221; 321; 421; 521 de l'organe de mesure. Ici, l'axe de mesure correspond alors à l'axe de révolution de la cavité conique 121; 221; 321; 421; 521.

Les axes de mesures AM1; AM2; AM3; AM4; AM5 des organes émetteurs-récepteurs 120; 220; 320; 420; 520; 620 sont ici parallèles entre eux.

En variante, on peut envisager que ces axes de mesure ne soit pas parallèles entre eux. Leur inclinaison peut permettre de prendre en compte une forte courbure de la monture de lunettes portée par l'individu.

Il est particulièrement avantageux d'utiliser un organe de mesure comportant une cavité conique au fond de laquelle est disposé un élément de mesure, car cette cavité conique joue un rôle de guide d'onde et contribue à une amplification du signal.

Plus précisément, chaque organe émetteur-récepteur 120; 220; 320; 420; 520; 620 comporte ici un élément émetteur-récepteur 122; 222; 322; 422; 522 placé à l'intérieur d'une cavité conique 121; 221; 321; 421; 521 d'angle au sommet A1; A2; A3; A4; A5 compris entre 15 et 45 degrés, à une profondeur H1; H2; H3; H4; H5 d'au moins 30 millimètres.

L'angle au sommet des cônes de mesure des organes émetteurs-récepteurs réalise un compromis entre une ouverture suffisamment grande pour émettre des ultrasons en direction de l'ensemble du visage de l'individu, de manière à couvrir facilement les lunettes de cet individu et une ouverture restreinte de manière à ne recevoir que les ultrasons réfléchis sur la tête de l'individu et principalement sur les lunettes de cet individu.

Le cône de mesure de l'organe de mesure désigne un angle solide qui représente l'ensemble des axes de propagation des ultrasons de l'espace selon lesquelles les ultrasons sont émis ou reçus par l'organe de mesure. Cet angle solide est centré sur cet axe de mesure et correspond sensiblement à l'angle solide délimité par la cavité conique.

Ces organes émetteurs-récepteurs 120; 220; 320; 420; 520 sont alignés selon une droite parallèle aux faces supérieure et inférieure du boîtier du support de mesure.

Plus précisément, les deux organes émetteurs-récepteurs sont ici distants de 7 centimètres. Cet écart permet de placer chaque organe émetteur-récepteur en vis-à-vis de l'une des lentilles ophtalmiques de la monture 30 de l'individu.

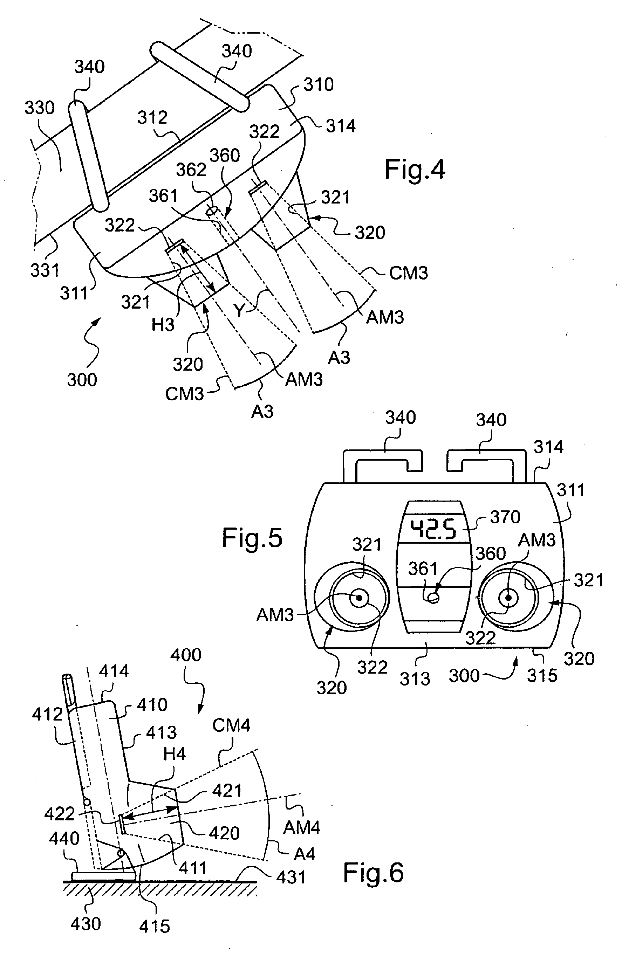

Selon un premier mode de réalisation du dispositif de mesure 100; 200; 300; 400 et ses variantes, celui-ci comporte en outre un support de lecture 130; 330; 430 présentant une partie d'affichage 131; 331; 431 plane adaptée à l'affichage de signes, comme représenté sur les figures 4, 6 et 7. Le dispositif de mesure 100; 200; 300; 400 comporte de plus des moyens 140; 240; 340; 440 pour le montage amovible du support de mesure 110; 210; 310; 410 sur le support de lecture 130; 330; 430.

La face arrière 112; 212; 312; 412 du boîtier 111; 211; 311; 411 du support de mesure 110; 210; 310; 410 est avantageusement plane afin de pouvoir monter facilement le support de mesure sur le support de lecture.

Dans l'exemple représenté sur la figure 7, le support de lecture 131 est constitué par un écran d'un ordinateur portable 132 dont l'écran 130 est articulé au clavier 133. Les moyens de montage amovible 140 sont adaptés à opérer un clipsage du support de mesure sur l'écran 130.

En variante, le support de lecture peut être un livre, un écran d'affichage autre qu'un écran d'ordinateur ou une tablette comportant des optotypes par exemple.

Les moyens de clipsage 140 du support de mesure représenté sur les figures 1 et 7 sont représentés plus particulièrement sur la figure 2 et consiste ici en un agencement de deux ressorts de torsion 140A reliés par un fil métallique rigide qui forme une patte d'accrochage s'étendant à partir de la face arrière 112 du boîtier 111 du support de mesure 110.

Les ressorts 140A sont ici déportés à distance de la face arrière 112 du boîtier 111 du support de mesure 110 de manière à autoriser la fixation du support de mesure 110 sur un support de lecture présentant une épaisseur importante.

Dans une première variante de ce premier mode de réalisation représentée sur la figure 3, les moyens de clipsage 240 comportent également une patte d'accrochage formée grâce à deux ressorts de torsion 240A. Ces ressorts de torsion 240 sont ici fixés à proximité de la face arrière 212 du boîtier 211 du support de mesure 210 et n'autorisent la fixation du support de mesure 210 que sur un support de lecture présentant une épaisseur plus faible que dans le cas du premier mode de réalisation de la figure 2.

Ce support de lecture peut être par exemple un écran d'ordinateur semblable à celui représenté sur la figure 7.

Selon une deuxième variante de ce premier mode de réalisation représentée sur les figures 4 et 5, lesdits moyens de montage comportent deux crochets 340 montés sur la face arrière 312 du boîtier 311 du support de mesure 310 et ouverts en direction de la face inférieure du support de mesure 300. Chaque crochet 340 comporte une branche support rectiligne montée à rotation sur la face arrière 312 du boîtier 311, de telle sorte que chaque crochet puisse au moins pivoter entre une position de repli dans laquelle il s'étend dans un plan sensiblement parallèle à la face arrière 312 du boîtier 311 du support de mesure 310 et une position de déploiement dans laquelle il s'étend dans un plan sensiblement perpendiculaire à la face arrière 312 du boîtier 311.

Chaque crochet 340 comporte également une branche centrale dont la longueur détermine l'épaisseur maximale du support de lecture 330 sur lequel le support de mesure peut être monté. Cette branche centrale comporte à son extrémité libre un retour qui permet l'accrochage sur le support de lecture 330.

Comme représenté sur la figure 4, les crochets 340 peuvent être adaptés à l'épaisseur du support de lecture 330 si celle-ci est inférieure à ladite épaisseur maximale par simple pivotement d'un angle approprié autour de la branche support. Ce support de lecture 330 peut être par exemple un écran d'ordinateur semblable à celui représenté sur la figure 7.

Selon une troisième variante du premier mode de réalisation, représentée sur la figure 6, le support de mesure 410 comporte un pied 440 permettant de poser le support de mesure 410 de façon stable sur un support de lecture 430.

Ce pied est ici rétractable : il pivote entre une position dans laquelle il est logé dans le boîtier 411 du support de mesure 410 et une position dans laquelle il est déployé hors du boîtier.

Le support de lecture 430 peut ici par exemple être une table sur laquelle une feuille comportant les signes à lire par l'individu est posée.

Enfin, selon une cinquième variante du premier mode de réalisation non représentée, le support de mesure peut être fixé sur un support quelconque qui n'est pas un support de lecture, par exemple sur un mur, et l'individu tient dans ses mains ou fixe du regard un support de lecture non lié mécaniquement au support de mesure.

De manière générale, le support de lecture 130; 330; 430 comporté en face avant, c'est-à-dire du côté destiné à être orienté vers la tête 10 de l'individu, une partie d'affichage 131; 331; 431 plane sur laquelle sont affichés des signes que l'individu fixe du regard lors de la mesure.

Les signes sont typiquement alignés suivant plusieurs lignes d'affichage et comportent par exemple des optotypes semblables à ceux utilisés par l'opticien pour évaluer le défaut visuel de l'individu. Il peut aussi s'agir d'un texte quelconque que l'individu lit.

Cette partie d'affichage 131; 331; 431 peut comporter des signes fixes ou un écran d'affichage dynamique autorisant l'affichage de différents signes, par exemple autorisant l'affichage de textes dans lesquels les lettres utilisées présentent différentes hauteurs, ou un texte dans différentes langues.

Le support de lecture 130; 330; 430 est de préférence portable et l'individu le tient entre ses mains pendant la mesure (voir figure 7).

Cependant, il peut être utile de rendre ce support de lecture inamovible, par exemple pour éviter le vol de ce support de lecture dans la boutique de l'opticien.

Dans ce cas, il est monté par exemple sur un bras permettant le réglage de sa position dans l'espace devant l'individu, notamment en ce qui concerne la distance entre le support de lecture et la tête de l'individu dans un plan horizontal et la hauteur entre le support de lecture et la tête de l'individu dans un plan vertical ainsi que l'inclinaison de ce support de lecture autour d'un axe horizontal.

L'individu peut alors régler à la main la position du support de lecture en faisant varier ces paramètres ou faire ces réglages de manière télécommandée, ou encore donner à un opérateur les instructions nécessaires pour que le support de lecture soit placée dans une position de lecture confortable pour cet individu.

De préférence, l'individu ajuste la position relative de sa tête par rapport au support de lecture pour se trouver dans une position naturelle et confortable de lecture.

Selon un deuxième mode de réalisation du dispositif selon l'invention, représenté sur les figures 8 à 10, le dispositif de mesure 500; 600 comporte un support de lecture 530 présentant une partie d'affichage 531 plane adaptée à l'affichage de signes, et des moyens pour le montage amovible du support de lecture 530 sur le support de mesure 510; 610. Le support de lecture 530 est par exemple un téléphone portable ou une tablette multimédia qui peut être fournie par l'opticien ou propre à l'individu.

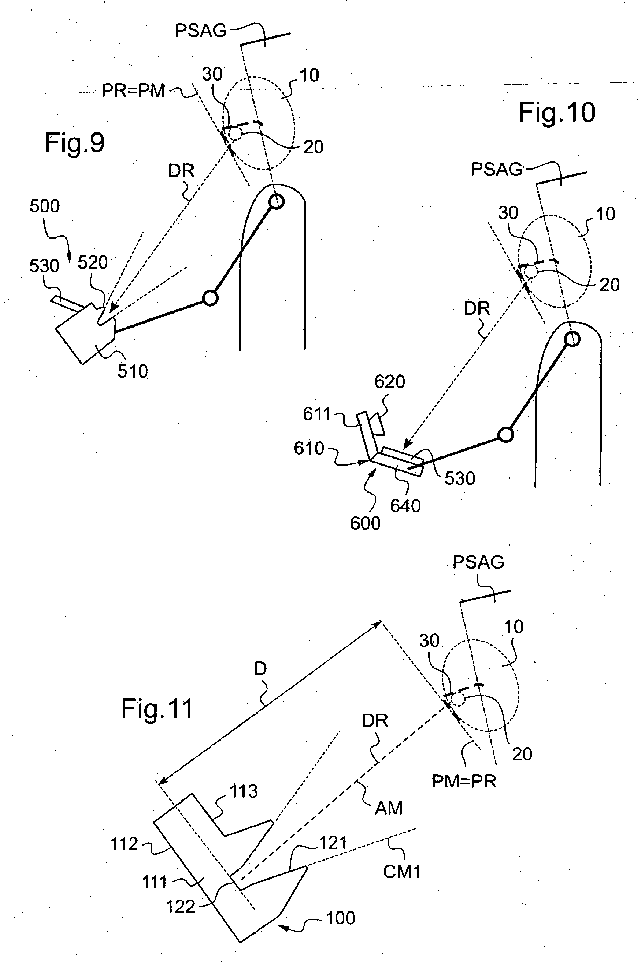

Dans une première variante de ce deuxième mode de réalisation, illustrée par les figures 8 et 9, le support de mesure 510 forme une station d'accueil du support de lecture 530.

Le boîtier 511 du support de mesure 510 forme alors une embase adaptée à reposer de façon stable sur un plan horizontal ou légèrement incliné, tel que le dessus d'une table, et comporte des moyens de raccordement mécanique adaptés à coopérer avec des moyens de raccordement mécanique complémentaires du support de lecture, de telle manière que, après raccordement, le plan de la partie d'affichage 531 du support de lecture 530 soit oblique par rapport au plan sur lequel repose l'embase 511, c'est-à-dire incliné d'un angle inférieur à 90 degrés par rapport à ce plan. Le boitier-embase 511 peut ainsi, en service, reposer sur une table ou être tenu à la main par l'individu pour un confort de lecture optimum, comme illustré par le figure 9.

Les moyens de raccordement mécanique comportent un logement 540 ménagé en renfoncement de la face supérieure horizontale du boîtier 511 et adapté à accueillir et à bloquer une partie inférieure du support de lecture 530. Le support de lecture 530 surplombe donc, lorsqu'il est raccordé, le boîtier 511 du support de mesure 510 et ses organes émetteurs-récepteurs 520.

Dans une deuxième variante de ce deuxième mode de réalisation, illustrée par la figure 10, le boîtier 611 du support de mesure 610 est porté par une embase 640 adaptée à reposer de façon stable sur un plan horizontal ou légèrement incliné, tel que le dessus d'une table, et comporte des moyens de raccordement mécanique adaptés à coopérer avec des moyens de raccordement mécanique complémentaires du support de lecture 530. Ces moyens de raccordement mécanique (non représentés) de l'embase 640 sont typiquement réalisés sous la forme de moyens de clipsage ou d'accueil ajusté ou serré du support de lecture 530.

Le boîtier 611 du support de mesure 610 forme un L avec l'embase 640, de telle sorte que les organes émetteurs-récepteurs 620 surplombent l'embase 640 et le support de lecture 530. Avantageusement, on peut prévoir que le boitier 611 soit articulé à l'embase 640 autour d'un axe horizontal.

En l'espèce, le support de lecture est accueilli à plat sur l'embase 640, de telle sorte que le plan de sa partie d'affichage 531 soit parallèle au plan sur lequel l'embase 640 est adaptée à reposer. L'embase 640 peut ainsi, en service, reposer sur une table ou être tenue à la main par l'individu pour un confort de lecture optimum, comme illustré par la figure 10.

Dans les deux variantes de ce deuxième mode de réalisation, le support de mesure 510; 610 peut également comporter des moyens de raccordement électroniques et informatiques avec le support de lecture 530. Le support de mesure 510; 610 peut alors communiquer avec le support de lecture 530. Il est alors possible de prévoir par exemple un ajustement de la taille des signes affichés par le support de lecture 530 en fonction de la distance de lecture mesurée par le support de mesure 510; 610.

Quel que soit le mode de réalisation du dispositif 100; 200; 300; 400; 500 de mesure selon l'invention, celui-ci comporte une unité de traitement électronique adaptée à délivrer, en fonction du signal d'ultrasons reçu par au moins l'un des organes émetteurs-récepteurs 120; 220; 320; 420; 520; 620, un signal représentatif de la distance D entre la monture 30 de lunette équipant la tête 10 de l'individu (figure 101 et le support de mesure 100; 200; 300; 400; 500 et à en déduire la distance caractéristique de lecture recherchée.

De préférence, l'unité de traitement électronique est adaptée à déduire la distance caractéristique de lecture recherchée des signaux délivrés par au moins l'un des deux organes émetteurs-récepteurs 120; 220; 320; 420; 520; 620, de préférence par les deux.

Le dispositif de mesure 100; 200; 300; 400; 500 comporte également des moyens pour vérifier que la monture 30 est au moins partiellement située dans le cône de mesure CM1; CM2; CM3; CM4; CM5 de l'organe émetteur-récepteur considéré.

Quel que soit le mode de réalisation du dispositif de mesure, les organes de mesure 120; 220; 320; 420; 520; 620 peuvent être, soit montés fixes sur ledit support de mesure 110; 210; 310; 410; 510, soit montés mobiles sur le support de mesure 110; 210; 310; 410; 510 pour au moins pivoter par rapport à un axe de pivotement perpendiculaire à leur axe de mesure AM1; AM2; AM3; AM4; AM5. Dans ce dernier cas, on peut envisager qu'un seul organe de mesure soit monté mobile sur le support de mesure.

Dans les exemples représentés sur les figures annexées, les organes émetteurs-récepteurs 120; 220; 320; 420 des dispositifs de mesure selon le premier mode de réalisation et ses variantes sont montés fixes sur le support de mesure 110; 210; 310; 410, tandis que les organes émetteurs-récepteurs 520; 620 du dispositif de mesure 500; 600 décrits en référence aux figures 8 à 10 pour les deux variantes du deuxième mode de réalisation sont montés mobiles sur le support de mesure 510; 610 pour pivoter autour de l'axe de pivotement X perpendiculaire aux axes de mesures AM5 de ces organes émetteurs-récepteurs 520; 620.

Dans le cas du premier mode de réalisation, les moyens pour vérifier que la tête de l'individu est au moins partiellement située dans le cône de mesure dudit organe de mesure principal comportent des moyens de détection visuelle 160; 360 permettant à l'individu de détecter visuellement une position relative de sa tête et de l'organe de mesure principal dans laquelle sa tête est au moins partiellement située dans le cône de mesure dudit organe de mesure principal.

Ces moyens de détection visuelle 160 comportent par exemple, comme représenté sur la figure 1, une cible 162 et un marquage 161 portés par le support de mesure 110 et solidaires des organes émetteurs-récepteurs 120, le marquage 161 et la cible 162 étant décalés suivant la direction commune des axes de mesure AM1 de ces organes émetteurs-récepteurs 120.

Cette cible 162 et ce marquage 161 son agencés de manière à ce qu'ils soient visuellement perçus par l'individu comme centrés l'un par rapport à l'autre uniquement lorsque la tête de l'individu est au moins partiellement située dans le cône de mesure dudit organe de mesure principal. Ceci est dû à un effet de parallaxe résultant du décalage du marquage 161 et de la cible 162 suivant la direction des axes de mesure AM1.

La cible 162 consiste ici en un cercle 162 qui se trouve dans un plan parallèle à la face avant 113 du support de mesure 110 orientée vers l'individu, en retrait par rapport à cette face avant 113. La cible 162 est visible par l'individu à travers une fenêtre transparente 163 du boîtier 111.

Le marquage 161 est ici par exemple un disque opaque qui est collé sur la fenêtre 163.

En variante, différents couples de cibles et marquages peuvent être utilisés : cercle au centre d'un carré, cercle entre deux lignes parallèles, ou deux cercles concentriques, par exemple.

Alternativement, comme représenté sur la figure 4, les moyens de détection visuelle comportent au moins une cible 362 appartenant au support de mesure et donc solidaire des organes émetteurs-récepteurs. Cette cible 362 est visible par l'individu uniquement lorsque la tête de l'individu est au moins partiellement située dans le cône de mesure dudit organe de mesure principal.

La cible 362 est de préférence une source lumineuse du type diode électroluminescente disposée au fond d'une cavité cylindrique 361 débouchant en face avant 313 du boîtier du support de mesure, de telle sorte qu'elle soit visible par l'individu uniquement lorsque la direction de regard DR de l'individu s'étend selon l'axe longitudinale Y de la cavité cylindrique 361, cette direction de regard correspondant à une position relative de la tête 10 de l'individu et du support de mesure 310 dans laquelle la tête de l'individu est au moins partiellement située dans le cône de mesure CM3 des organes émetteurs-récepteurs.

En variante la cible est un signe affiché par un écran d'affichage et visible seulement sous un angle d'incidence du regard de l'individu tel que lorsque l'individu regarde la cible sous cet angle d'incidence, la tête de l'individu est au moins partiellement située dans le cône de mesure dudit organe de mesure principal.

En pratique, pour mettre en oeuvre ces variantes du premier mode de réalisation, l'individu place le support de mesure 110; 210; 310; 410 sur son support de lecture 130; 330; 430 et se place dans une position de lecture naturelle pour lire les signes affichés sur la partie d'affichage du support de lecture.

Il regarde ensuite en direction du support de mesure 110; 210; 310; 410 sans déplacer la tête et ajuste la position de celui-ci par rapport au support de lecture, puis éventuellement la position du support de lecture, afin de centrer la cible 162 et le marquage 161 dans le cas du dispositif de mesure 100 représenté sur les figures 1 et 7, ou afin de visualiser la cible 362 lumineuse dans le cas du dispositif de mesure 300 représenté sur les figures 4 et 5.

La mesure de la distance D entre le support de mesure et les lunettes de l'individu est ensuite réalisée. Cette étape sera décrite plus en détail ultérieurement.

L'individu reporte enfin son regard sur la partie d'affichage du support de lecture. En variante, on peut avantageusement prévoir que la mesure de la distance D soit réalisée après que l'individu a reporté son regard sur la partie d'affichage du support de lecture.

Dans le cas du deuxième mode de réalisation, les moyens pour vérifier que la tête 10 de l'individu est au moins partiellement située dans le cône de mesure CM5 d'au moins un des organes émetteurs-récepteurs 520 comportent des moyens de détection automatique pour détecter automatiquement une position relative de la tête de l'individu et de l'organe émetteur-récepteur 520 en question dans laquelle sa tête est au moins partiellement située dans le cône de mesure CM5 considéré.

Les organes émetteurs-récepteurs 520 sont ici montés pour pivoter conjointement sur le support de mesure 510. Ces moyens de détection automatique comportent ainsi des moyens de balayage d'une plage de positions angulaires de ces organes émetteurs-récepteurs 520 par rapport au support de mesure 510, et des moyens de détermination de la position angulaire de ces organes émetteurs-récepteurs 520 correspondant à un signal d'ultrasons reçu, maximal ou supérieur à un seuil.

Pour cela, les moyens de balayage font pivoter, pendant l'émission d'ultrasons, les organes émetteurs-récepteurs 520 pour que l'axe de mesure AM5 de chaque organe émetteur-récepteur 520 balaye la tête 10 de l'individu. La plage de positions angulaires balayée couvre au moins 15 degrés vers l'avant ou vers l'arrière du support de mesure 510 par rapport à une position angulaire moyenne de référence dans laquelle l'angle GAMMA, formé entre l'axe de mesure de l'organe récepteur et la face avant 513 du boîtier 511 du support de mesure 510, est compris entre 10 et 80 degrés (figure 8).

Le balayage est de préférence réalisé de manière automatique : le pivotement des organes de mesure 520 est alors motorisé et commandé par des moyens de pilotage électronique.

Ce balayage peut également être réalisé manuellement par l'individu. Le support de mesure comporte alors des moyens de pivotement manuel tel qu'une molette.

Un balayage automatique présente l'avantage de ne pas solliciter l'individu.

Ladite unité de traitement électronique traite les signaux ultrasons détectés en fonction de la position angulaire des organes émetteurs-récepteurs 520 et en déduit la position angulaire de mesure recherchée.

En particulier, la position angulaire de mesure correspond à celle pour laquelle une intensité maximale de signaux ultrasons est détectée.

Alternativement, la position angulaire de mesure correspond à celle pour laquelle l'intensité du signal ultrasons est supérieure à un seuil.

Ce seuil peut correspondre par exemple à un pourcentage de l'intensité maximale du signal d'ultrasons obtenue lors d'une étape de calibration.

La durée d'émission d'ultrasons au bout de laquelle l'intensité maximale de signal est détectée est en générale inférieure ou égale à 40 secondes.

Les moyens de pilotage électroniques des moyens de balayage motorisés commandent alors le pivotement des organes émetteurs-récepteurs 520 vers cette position angulaire.

Une fois que les organes émetteurs-récepteurs sont dans la position angulaire de mesure, l'émission d'ultrasons est déclenchée et le signal d'ultrasons est détecté.

La mesure est ainsi rapide et aisée à réaliser.

Dans le cas d'un balayage manuel, un signal sonore ou lumineux lui indique alors par exemple que la position angulaire de mesure est atteinte.

En pratique, pour mettre en oeuvre ce deuxième mode de réalisation, l'individu place le support de lecture 530 sur le support de mesure 510 et se place dans une position de lecture naturelle pour lire les signes affichés sur la partie d'affichage du support de lecture 530.

Il réalise ensuite l'étape de balayage automatique ou manuel de la plage angulaire des organes émetteurs-récepteurs 520. La position angulaire recherchée est déterminée par l'unité de traitement électronique et les organes émetteurs-récepteurs 520 sont amenés soit automatiquement dans cette position, soit manuellement par l'individu.

La mesure de la distance D entre le support de mesure et les lunettes de l'individu est alors réalisée.

Selon un troisième mode de réalisation dérivé du premier mode de réalisation, il est possible de prévoir un montage du support de mesure sur le support de lecture avec un degré de liberté en pivotement autour d'un axe perpendiculaire aux axes de mesure des organes émetteurs-récepteurs. Le support de mesure est alors par exemple monté à pivotement sur une pièce intermédiaire comportant des moyens de clipsage ou des crochets pour son montage amovible sur le support de lecture.

Les moyens pour vérifier que la tête de l'individu est au moins partiellement située dans le cône de mesure dudit organe de mesure principal peuvent alors comporter des moyens de détection automatique comprenant des moyens de balayage d'une plage de positions angulaires des organes émetteurs-récepteurs par rapport au support de mesure, ce balayage étant réalisé soit manuellement par l'individu qui fait pivoter ce support de mesure en agissant directement sur lui, soit automatiquement si le pivotement du support de mesure est motorisé. Ces moyens de détection automatique comprennent également des moyens de détermination de la position angulaire de mesure de ces organes émetteurs-récepteurs correspondant à un signal d'ultrasons reçu, maximal ou supérieur à un seuil.

Pour cela, les moyens de balayage font pivoter, pendant l'émission d'ultrasons, le support de mesure pour que l'axe de mesure de chaque organe émetteur-récepteur balaye la tête de l'individu. La plage de positions angulaires balayée couvre au moins 15 degrés vers l'avant ou vers l'arrière du support de mesure par rapport à une position angulaire moyenne de référence dans laquelle l'angle entre l'axe de mesure de l'organe récepteur et la partie d'affichage du support de lecture est compris entre 10 et 80 degrés.

Le balayage est de préférence réalisé de manière automatique : le pivotement est alors motorisé et commandé par des moyens de pilotage électronique.

Le fonctionnement du dispositif de mesure reste similaire à celui décrit pour le deuxième mode de réalisation.

De manière générale, les moyens pour vérifier que la tête de l'individu est au moins partiellement située dans le cône de mesure d'au moins un des organes émetteurs-récepteurs sont de préférence agencés pour vérifier que l'axe de mesure de cet organe émetteur-récepteur est sensiblement perpendiculaire au plan PM de la monture portée par l'individu. Dans cette configuration, le signal d'ultrasons réfléchi sur la monture et les lentilles ophtalmiques et reçu par l'organe émetteur-récepteur en question est maximal et la mesure est ainsi plus précise.

Quel que soit le mode de réalisation du dispositif de mesure, l'unité de traitement électronique du signal d'ultrason reçu par les organes émetteurs-récepteurs du dispositif de mesure 100; 200; 300; 400; 500 est adaptée à délivrer, en fonction du signal reçu par les organes émetteurs-récepteurs, un signal représentatif de la distance entre la monture 30 (comme dans l'exemple décrit) ou les lentilles (en variante analogue aisément transposable) des lunettes équipant la tête 10 de l'individu et le support de mesure 110; 210; 310; 410; 510 et en déduire ladite distance caractéristique de lecture.

L'unité de traitement détermine tout d'abord la distance D entre la monture 30 de lunettes de l'individu et le support de mesure 110; 210; 310; 410; 510, ci-après distance de mesure D.

L'unité de traitement électronique est adaptée à extraire du signal d'ultrasons reçu par l'organe récepteur la partie de ce signal qui est réfléchie par les lentilles ou la monture des lunettes portées par l'individu, à l'exclusion de la partie de ce signal qui est réfléchie par la peau de l'individu.

L'intensité de signal reçu dépend de la nature du matériau ayant réfléchi le signal d'ultrasons émis et de l'écart entre la position angulaire de l'axe de mesure des organes émetteurs-récepteurs et la position angulaire de mesure dans laquelle cet axe de mesure est perpendiculaire au plan moyen de la monture. La position angulaire de mesure correspond ainsi à un signal d'ultrasons reçu maximal.

L'amplitude absolue du signal reçu, c'est-à-dire son intensité, permet de déduire si le signal reçu provient d'une réflexion sur la peau de l'individu ou d'une réflexion sur la monture de lunettes.

A titre d'exemple, pour une distance D d'environ 40 centimètres, le signal reçu après réflexion sur la monture est environ quatre fois plus intense que le signal reçu après réflexion sur la peau.

Il est ainsi possible de discriminer le signal reçu après réflexion sur la monture de lunettes du signal reçu après réflexion sur la peau, et ce même si la position angulaire de l'axe de mesure des organes émetteurs-récepteurs n'est pas exactement la position angulaire de mesure.

L'unité de traitement électronique du dispositif de mesure réalise un ajustement automatique du gain, c'est-à-dire de l'amplification du signal reçu par l'élément récepteur ou émetteur récepteur, en fonction de l'amplitude du signal reçu.

Cette amplitude dépend notamment de la surface des lunettes portées par l'individu. Une petite monture de lunettes donnera un signal reçu d'amplitude plus faible qu'une monture de lunettes de plus grande taille.

L'augmentation de l'amplification de ce signal par l'unité de traitement électronique du dispositif de mesure permet de compenser cette différence.

En outre, en cas de pic de signal très intense, d'amplitude supérieure à une première valeur seuil prédéfinie, l'unité de traitement électronique est programmée pour procéder à un écrêtement du signal avant de réaliser l'ajustement du gain.

L'unité de traitement électronique du dispositif de mesure détecte le signal reçu dont l'intensité est supérieure à une deuxième valeur seuil prédéterminée. Cette deuxième valeur seuil correspond de préférence à une valeur d'intensité du signal assurant que le signal reçu provient d'une réflexion du signal émis sur la monture de lunettes de l'individu.

Cette deuxième valeur seuil est déterminée à partir de mesures d'étalonnage réalisées au préalable ou correspond à une valeur standard prédéterminée par rapport au signal maximal attendu pour une monture de taille donnée, et pour un gain déterminé. Cette valeur standard est par exemple prise égale à 20 pour cent de ce signal maximal.

Plusieurs valeurs seuil inférieures à la deuxième valeur seuil peuvent être prévues pour prendre en compte la diminution de l'intensité du signal reçu en fonction de l'écart entre la position angulaire de l'axe de mesure des organes émetteurs-récepteurs et la position angulaire de mesure.

Ces différents seuils sont détectés successivement par l'unité de traitement électronique du dispositif de mesure en cas d'échec de détection d'un signal au-dessus de la deuxième valeur seuil. Les autres valeurs seuil sont détectées selon un ordre décroissant jusqu'à ce qu'un signal soit détecté.

Ce mode de seuillage permet d'éviter les échecs de détection, qui obligeraient à réaliser une nouvelle mesure.

Si le signal reçu présente une intensité inférieure à la valeur seuil la plus basse, le dispositif de mesure indique par un message d'erreur que la mesure doit être réitérée.

Le signal reçu en fonction du temps présenterait, dans un cas idéal où l'élément récepteur ne recevrait que le signal réfléchi par les lunettes de l'individu, une forme carrée.

En réalité, le signal d'ultrasons reçu après réflexion sur la tête du porteur comporte une composante aux temps les plus courts correspondant au signal réfléchi par la peau de l'individu située en avant des lunettes, puis une composante à des temps intermédiaires correspondant au signal réfléchi par les lunettes de l'individu et enfin une composante aux temps longs correspondant au signal réfléchi par la peau de l'individu située en arrière des lunettes par rapport au support de mesure.

Le signal reçu par les organes récepteurs ou émetteurs-récepteurs présente alors une forme approchée aplatie qui est liée à la texture de la surface sur laquelle le signal ultrasonore est réfléchi.

Les moyens de traitement du signal reçu procèdent alors à une étape de détection du signal réfléchi par la monture ou les lentilles correctrices des lunettes portées par l'individu. Ils opèrent à cet effet une correction de la pente du signal reçu réel par amplification de cette pente afin de se rapprocher du cas idéal et déterminer précisément le moment auquel le signal réfléchi sur les lunettes de l'individu est reçu.

Ce moment est par exemple déterminé comme le temps correspondant à un seuil paramétrable (typiquement situé entre la mi-hauteur et 20% de la hauteur) de la pente ascendante du signal reçu.

Une première valeur de la distance de mesure séparant les lunettes de l'individu et les organes émetteur et récepteur du support de mesure est alors déterminée en fonction de ce temps.

De préférence, une fois une première valeur de la distance de mesure déterminée, le signal reçu subit également une étape de correction d'étalonnage correspondant à la soustraction d'un signal de référence correspondant au signal reçu lorsque les ultrasons sont réfléchis par la peau.

Ce signal de référence correspond au signal reçu par l'organe récepteur ou émetteur-récepteur par réflexion sur la peau du visage d'un individu de référence moyen lorsque cet individu de référence se trouve à une distance du dispositif de mesure égale à la première valeur de la distance de mesure.

Ce signal de référence est par exemple déterminé en réalisant trois mesures d'étalonnage à des distances de mesure connues, par exemple pour des distances de mesure de 30, 40 et 50 centimètres.

Une interpolation linéaire de ces signaux permet d'un déduire un signal de référence pour toutes les distances de mesure comprises entre 30 et 50 centimètres.

L'unité de traitement électronique du signal soustrait au signal reçu le signal de référence correspondant à la première valeur de la distance de mesure déterminée.

Les étapes précédentes sont ensuite répétées sur ce signal corrigé afin de déterminer une deuxième valeur de cette distance de mesure plus précise que la première.

La précision de la distance de mesure ainsi déterminée est d'environ 1 à 2 centimètres.

La durée totale de la mesure est d'environ 1 minute. Cette mesure est donc réalisée très rapidement.

Plus particulièrement ici, une première distance de mesure moyenne entre le support de mesure et les lunettes de l'individu est déterminée à partir de la moyenne des signaux d'ultrasons détectés par les deux organes émetteurs-récepteurs 120; 220; 320; 420; 520 du support de mesure 110; 210; 310; 410; 510 et qui est traitée par l'unité de traitement électronique comme expliqué précédemment.

Les deux organes émetteurs-récepteurs du support de mesure sont disposés chacune en regard de l'une des lentilles ophtalmiques de la monture 30 de lunettes de l'individu, et donne chacun une mesure de la distance de mesure entre l'une des lentilles ophtalmiques de cette monture 30 et l'organe émetteur-récepteur correspondant.

La première distance de mesure moyenne entre le support de mesure et les lunettes correspond donc à une moyenne des distances entre chaque lentille et le support de mesure.

La réalisation de cette moyenne permet de limiter l'influence sur la mesure d'une faible rotation de la tête autour d'un axe verticale : cela limite l'erreur introduite par le fait que le plan sagittal PSAG de la tête du porteur n'est pas parfaitement confondu avec le plan de symétrie PS du dispositif de mesure.

De préférence, les mesures sont répétées et moyennées : la distance entre le support de mesure et les lunettes est déterminée à partir de la moyenne d'au moins cinq premières distances de mesure moyenne ainsi déterminées.

En variante, l'unité de traitement électronique détermine deux distances entre le support de mesure et les lunettes à partir des signaux d'ultrasons reçus par chacun des deux organes émetteurs-récepteurs qui correspondent respectivement à la mesure de la distance séparant chaque lentille ophtalmique du support de mesure. L'unité de traitement électronique fait ensuite la moyenne de ces deux distances pour déterminer une valeur de la distance entre le support de mesure et les lunettes recherchée. Plusieurs de ces valeurs peuvent ensuite être moyennées.

La mesure est ainsi plus précise.

L'unité de traitement électronique déduit ensuite de cette distance de mesure la distance caractéristique de lecture de l'individu recherchée.

Pour cela, l'unité de traitement corrige la distance entre le support de mesure et les lunettes déterminée précédemment en fonction de la position relative du support de mesure et du support de lecture.

Selon une première possibilité, le dispositif de mesure comporte des moyens mécaniques pour figer la position relative du support de lecture par rapport au support de mesure et l'unité de traitement électronique est adaptée à calculer ladite distance caractéristique de lecture en fonction de cette position figée.

On peut par exemple envisager que la position relative du support de mesure et du support de lecture soit prédéterminée et fixe. C'est le cas par exemple du deuxième mode de réalisation du dispositif représenté sur la figure 8.

C'est également le cas dans le premier mode de réalisation du dispositif représenté sur les figures 1 à 6 si le support de mesure est toujours placé sur le support de lecture à une même distance de la partie d'affichage de ce support de lecture. Les moyens mécaniques pour figer la position relative du support de lecture par rapport au support de mesure peuvent alors être par exemple des moyens de butée disposés sur le support de lecture pour limiter les positions dans lesquelles le support de mesure peut être monté sur le support de lecture.

Dans ce cas, il est possible de mettre en mémoire dans l'unité de traitement électronique les paramètres de position relative tels que la distance et l'inclinaison relative du support de lecture et du support de mesure. L'unité de mesure est alors programmée pour déterminer la distance caractéristique de lecture recherchée, qui correspond à la distance entre les lunettes et le support de lecture, en fonction de la distance entre les lunettes et le support de mesure et en fonction de ces paramètres.

On peut également envisager que la position relative du support de mesure et du support de lecture soit réglable. Le dispositif de mesure comporte alors des moyens pour déterminer la position relative du support de lecture par rapport au support de mesure suivant une direction de mesure.

Ceux-ci comportent notamment par exemple des moyens pour mesurer un angle d'inclinaison du support de mesure par rapport à l'horizontale et des moyens pour mesurer la distance entre le support de mesure et le support de lecture. Il s'agit par exemple d'au moins un inclinomètre embarqué sur le support de mesure. On peut également prévoir un deuxième inclinomètre embarqué sur le support de lecture.

On peut également envisager, dans le cas où le support de mesure est monté sur un support différent du support de lecture, par exemple sur un mur, que l'unité de traitement électronique soit programmée pour déduire du signal d'ultrasons reçu la distance entre les lunettes et le support de mesure, ainsi que la distance entre le support de lecture et le support de mesure, par un traitement similaire à celui décrit ci-dessus.

L'unité de traitement électronique peut alors corriger la distance entre les lunettes et le support de mesure déterminée en fonction de la distance entre le support de lecture et le support de mesure, pour en déduire la distance entre les lunettes et le support de lecture recherchée.

Le support de mesure comporte en outre éventuellement des moyens de transmission de la mesure de distance réalisée, avec ou sans fil, vers un ou plusieurs autres dispositifs utilisés par l'opticien, en particulier vers le support de lecture.

L'unité de traitement électronique peut alors par exemple être adaptée à piloter le support de lecture pour modifier la taille des signes affichés en fonction de la distance caractéristique de lecture déterminée.

Le support de mesure peut en particulier être une unité de mesure faisant partie d'un ensemble de mesure plus important.

Le support de mesure peut également comporter des moyens d'affichage 170; 370; 570 de la distance caractéristique de lecture déterminée, comme représenté sur les figures 1, 5 et 8. Celle-ci peut alors être lue directement sur le dispositif de mesure par l'individu ou par l'opticien.

Le support de mesure 110; 210; 310; 410; 510 comporte de préférence des moyens d'alimentation autonomes, par exemple des piles ou une batterie rechargeable.

En variante les moyens pour vérifier que cette monture de lunettes est au moins partiellement située dans le cône de mesure dudit organe de mesure principal peuvent aussi comporter un miroir disposé sur la face avant du support de mesure et dans lequel l'individu doit pouvoir visualiser ses yeux.

## Revendications

1. Dispositif (100; 200; 300; 400; 500; 600) de mesure d'une distance caractéristique de lecture d'un individu portant une monture de lunettes (30) munie de lentilles ophthalmiques, comportant un support de mesure (110; 210; 310; 410; 510; 610) portant au moins un organe émetteur (120; 220; 320; 420 ;520; 620) et au moins un organe récepteur d'ultrasons (120; 220; 320; 420 ;520; 620), l'un au moins de ces organes, appelé organe de mesure principal (120; 220; 320; 420 ;520; 620), possédant un axe de mesure (AM1; AM2; AM3; AM4; AM5), selon lequel l'émission ou la réception par l'organe de mesure principal (120; 220; 320; 420 ;520; 620) est privilégiée, et étant adapté à émettre ou à recevoir les ultrasons se propageant dans un cône de mesure (CM1; CM2; CM3; CM4; CM5) centré sur son axe de mesure (AM1; AM2; AM3; AM4; AM5), le dispositif comportant en outre une unité de traitement électronique adaptée à extraire du signal d'ultrasons reçu par l'organe récepteur (120; 220; 320; 420 ;520; 620) la partie de ce signal qui est réfléchie par les lentille ou la monture de lunettes portées par l'individu, à l'exclusion de la partie de ce signal qui est réfléchie par la peau de l'individu, de manière à délivrer, en fonction du signal d'ultrasons reçu par l'organe récepteur (120; 220; 320; 420 ;520; 620), un signal représentatif de la distance (D) entre la monture (30) de lunettes équipant la tête (10) de l'individu et le support de mesure (110; 210; 310; 410; 510) et en déduire ladite distance caractéristique de lecture, et des moyens (160; 360) pour vérifier que cette monture de lunettes est au moins partiellement située dans le cône de mesure dudit organe de mesure principal.

2. Dispositif (100; 200; 300; 400) selon la revendication 1, comportant un support de lecture présentant une partie d'affichage plane adaptée à l'affichage de signes et dans lequel l'unité de traitement électronique est adaptée à calculer ladite distance caractéristique de lecture comme la distance entre la monture (30) de lunettes équipant la tête (10) de l'individu et ledit support de lecture (130 ; 530).

3. Dispositif (100; 200; 300; 400) selon la revendication 2, comportant des moyens (140; 240; 340; 440) pour le montage amovible du support de mesure sur le support de lecture.

4. Dispositif (100) selon la revendication 3, dans lequel le support de lecture (130) est constitué par un écran d'ordinateur et les moyens de montage (140) amovible sont adaptés à opérer un clipsage du support de mesure (110) sur l'écran d'ordinateur (130).

5. Dispositif (500; 600) selon la revendication 2, comportant des moyens (540) pour le montage amovible du support de lecture (530) sur le support de mesure (510; 610, 640).

6. Dispositif (500; 600) selon la revendication 5, dans lequel ledit support de mesure (510; 610; 640) forme une station d'accueil du support de lecture (530), comportant une embase (511; 640) adaptée à reposer de façon stable sur un plan et des moyens de raccordement mécanique (540) amovibles adaptés à coopérer avec des moyens de raccordement complémentaires du support de lecture.

7. Dispositif selon l'une des revendications 1 à 6, dans lequel ledit organe de mesure principal (120; 220; 320; 420) est monté fixe sur ledit support de mesure (110, 210, 310, 410).

8. Dispositif (500) selon l'une des revendications 1 à 6, dans lequel ledit organe de mesure principal (520) est monté mobile sur ledit support de mesure (510) pour au moins pivoter par rapport à un axe (X) de pivotement perpendiculaire à l'axe de mesure (AM5) de cet organe de mesure principal (520).

9. Dispositif (100; 200; 300; 400) selon l'une des revendications 1 à 8, dans lequel les moyens (160; 360) pour vérifier que la tête de l'individu est au moins partiellement située dans le cône de mesuré (CM1; CM2; CM3; CM4) dudit organe de mesure principal (120; 220; 320; 420; 520) comportent des moyens de détection visuelle (161, 162; 361; 362) opérant selon le principe de la parallaxe pour permettre à l'individu de détecter visuellement une position relative de sa tête et de l'organe de mesure principal dans laquelle sa tête est au moins partiellement située dans le cône de mesure dudit organe de mesure principal.

10. Dispositif (500) selon la revendication 8, dans lequel les moyens pour vérifier que la tête de l'individu est au moins partiellement située dans le cône de mesure dudit organe de mesure principal comportent des moyens de détection automatique pour détecter automatiquement une position relative de la tête (10) de l'individu et dudit organe de mesure principal (520) dans laquelle sa tête (10) est au moins partiellement située dans le cône de mesure (CM5) dudit organe de mesure principal (520), lesdits moyens de détection automatique comportant des moyens de balayage d'une plage de positions angulaires dudit organe de mesure principal (520) par rapport au support de mesure, et des moyens de détermination de la position angulaire dudit organe de mesure principal (520) correspondant à un signal d'ultrasons reçu, maximal ou supérieur à un seuil.

11. Dispositif (100; 200; 300; 400; 500; 600) selon la revendication 1, comportant un support de lecture (130; 330; 430; 530) présentant une partie d'affichage (131; 331; 431; 531) plane adaptée à l'affichage de signes et des moyens mécaniques pour figer la position relative du support de lecture (130; 330; 430; 530) par rapport au support de mesure et dans lequel l'unité de traitement électronique est adaptée à calculer ladite distance caractéristique de lecture en fonction de cette position figée.

12. Dispositif (100; 200; 300; 400) selon la revendication 1, comportant un support de lecture (130; 330;430) présentant une partie d'affichage (131; 331; 431) plane adaptée à l'affichage de signes et des moyens pour déterminer la position relative du support de lecture par rapport au support de mesure suivant une direction de mesure.

13. Dispositif selon la revendication 12, dans lequel les moyens pour déterminer la position relative du support de lecture comportent des moyens pour mesurer un angle d'inclinaison du support de mesure par rapport à l'horizontale et des moyens pour mesurer la distance entre le support de mesure et le support de lecture.

14. Dispositif (100; 200; 300; 400; 500; 600) selon la revendication 1, comportant un support de lecture (130; 330; 430; 530) présentant une partie d'affichage (131; 331; 431; 531) plane adaptée à l'affichage de signes et une unité de traitement électronique adaptée à piloter le support de lecture pour modifier la taille des signes affichés en fonction de la distance caractéristique de lecture déterminée.

15. Dispositif (100; 200; 300; 400; 500; 600) selon l'une des revendications 1 à 14, dans lequel l'organe récepteur (120; 220; 320; 420; 520; 620) comporte un élément récepteur (122; 222; 322; 422; 522) placé à l'intérieur d'une cavité conique (121; 221; 321; 421; 521) d'angle au sommet (A1; A2; A3; A4; A5) compris entre 15 et 45 degrés, à une profondeur (H1; H2; H3; H4. H5) d'au moins 30 millimètres.

## Patentansprüche

1. Vorrichtung (100; 200; 300; 400; 500; 600) zur Messung eines charakteristischen Leseabstands einer Person, die eine Brillenfassung (30) trägt, die mit ophtalmologischen Linsen versehen ist, umfassend einen Messträger (110; 210; 310; 410; 510; 610), der mindestens ein Sendeelement (120; 220; 320; 420; 520; 620) und mindestens ein Ultraschallempfangselement (120; 220; 320; 420; 520; 620) trägt, wobei mindestens eines dieser Elemente, Hauptmesselement (120; 220; 320; 420; 520; 620) genannt, eine Messachse (AM1; AM2; AM3; AM4; AM5) besitzt, entlang der das Senden oder Empfangen durch das Hauptmesselement (120; 220; 320; 420; 520; 620) bevorzugt ist, und geeignet ist, die Ultraschalle, die sich in einem Messkegel (CM1; CM2; CM3; CM4; CM5), der auf seine Messachse (AM1; AM2; AM3; AM4; AM5) zentriert ist, ausbreiten, zu entsenden oder zu empfangen, wobei die Vorrichtung ferner eine elektronische Verarbeitungseinheit umfasst, die geeignet ist, aus dem vom Empfangselement (120; 220; 320; 420; 520; 620) empfangenen Ultraschallsignal den Teil dieses Signals zu extrahieren, der von den Linsen oder der Brillenfassung, die von der Person getragen wird, reflektiert wird, mit Ausnahme des Teils dieses Signals, das von der Haut der Person reflektiert wird, um in Abhängigkeit von dem vom Empfangselement (120; 220; 320; 420; 520; 620) empfangenen Ultraschallsignal ein Signal zu liefern, das für den Abstand (D) zwischen der Brillenfassung (30), die am Kopf (10) der Person sitzt, und dem Messträger (110; 210; 310; 410; 510) repräsentativ ist, und davon den charakteristischen Leseabstand abzuleiten, und Mittel (160; 360), um zu prüfen, ob diese Brillenfassung zumindest teilweise in dem Messkegel des Hauptmesselements angeordnet ist.

2. Vorrichtung (100; 200; 300; 400) nach Anspruch 1, umfassend einen Leseträger, der einen flachen Anzeigeteil aufweist, der an die Anzeige von Zeichen angepasst ist, und bei der die elektronische Verarbeitungseinheit geeignet ist, den charakteristischen Leseabstand als den Abstand zwischen der Brillenfassung (30), die am Kopf (10) der Person sitzt, und dem Leseträger (130; 530) zu berechnen.

3. Vorrichtung (100; 200; 300; 400) nach Anspruch 2, umfassend Mittel (140; 240; 340; 440) zur abnehmbaren Montage des Messträgers auf dem Leseträger.

4. Vorrichtung (100) nach Anspruch 3, bei der der Leseträger (130) von einem Computerbildschirm gebildet sind, und die Mittel zur abnehmbaren Montage (140) dazu vorgesehen sind, den Messträger (110) auf den Computerbildschirm (130) zu clipsen.

5. Vorrichtung (500; 600) nach Anspruch 2, umfassend Mittel (540) zur abnehmbaren Montage des Leseträgers (530) auf dem Messträger (510; 610; 640).

6. Vorrichtung (500; 600) nach Anspruch 5, bei der der Messträger (510; 610; 640) eine Aufnahmestation des Leseträgers (530) bildet, umfassend einen Sockel (511; 640), der dazu vorgesehen ist, stabil auf einer Ebene zu stehen, und abnehmbare mechanische Anschlussmittel (540), die geeignet sind, mit komplementären Anschlussmitteln des Leseträgers zusammenzuwirken.

7. Vorrichtung nach einem der Ansprüche 1 bis 6, bei der das Hauptmesselement (120; 220; 320; 420) fest auf dem Messträger (110; 210; 310; 410) montiert ist.

8. Vorrichtung (500) nach einem der Ansprüche 1 bis 6, bei der das Hauptmesselement (520) beweglich auf dem Messträger (510) montiert ist, um zumindest in Bezug zu einer Schwenkachse (X) senkrecht auf die Messachse (AM5) dieses Hauptmesselements (520) zu schwenken.

9. Vorrichtung (100; 200; 300; 400) nach einem der Ansprüche 1 bis 8, bei der die Mittel (160; 360) zum Prüfen, ob sich der Kopf der Person zumindest teilweise in dem Messkegel (CM1; CM2; CM3; CM4) des Hauptmesselements (120; 220; 320; 420; 520) befindet, visuelle Erfassungsmittel (161; 162; 361; 362) umfassen, die nach dem Parallaxe-Prinzip wirken, um es der Person zu ermöglichen, visuell eine relative Position ihres Kopfes und des Hauptmesselements zu erfassen, in der sich ihr Kopf zumindest teilweise in dem Messkegel des Hauptmesselements befindet.

10. Vorrichtung (500) nach Anspruch 8, bei der die Mittel zum Prüfen, ob sich der Kopf der Person zumindest teilweise in dem Messkegel des Hauptmesselements befindet, automatische Erfassungsmittel umfassen, um automatisch eine relative Position des Kopfes (10) der Person und des Hauptmesselements (520) zu erfassen, in der sich ihr Kopf (10) zumindest teilweise in dem Messkegel (CM5) des Hauptmesselements (520) befindet, wobei die automatischen Erfassungsmittel Mittel zum Abtasten eines Winkelpositionsbereichs des Hauptmesselements (520) in Bezug zum Messträger und Mittel zur Bestimmung der Winkelposition des Hauptmesselements (520) entsprechend einem empfangenen Ultraschallsignal, das maximal oder größer als eine Schwelle ist, umfassen.

11. Vorrichtung (100; 200; 300; 400; 500; 600) nach Anspruch 1, umfassend einen Leseträger (130; 330; 430; 530), der einen flachen Anzeigeteil (131; 331; 431; 531), der für die Anzeige von Zeichen geeignet ist, und mechanische Mittel aufweist, um die relative Position des Leseträgers (130; 330; 430; 530) in Bezug zum Messträger zu fixieren, und bei der die elektronische Verarbeitungseinheit geeignet ist, den charakteristischen Leseabstand in Abhängigkeit von dieser fixierten Position zu berechnen.

12. Vorrichtung (100; 200; 300; 400) nach Anspruch 1, umfassend einen Leseträger (130; 330; 430), der einen flachen Anzeigeteil (131; 331; 431), der für die Anzeige von Zeichen geeignet ist, und Mittel aufweist, um die relative Position des Leseträgers in Bezug zum Messträger in einer Messrichtung zu bestimmen.

13. Vorrichtung nach Anspruch 12, bei der die Mittel zur Bestimmung der relativen Position des Leseträgers Mittel, um einen Neigungswinkel des Messträgers in Bezug zur Horizontalen zu messen, und Mittel, um den Abstand zwischen dem Messträger und dem Leseträger zu messen, umfassen.

14. Vorrichtung (100; 200; 300; 400; 500; 600) nach Anspruch 1, umfassend einen Leseträger (130; 330; 430; 530), der einen flachen Anzeigeteil (131; 331; 431; 531), der für die Anzeige von Zeichen geeignet ist, und eine elektronische Verarbeitungseinheit aufweist, die geeignet ist, den Leseträger zu steuern, um die Größe der angezeigten Zeichen in Abhängigkeit von dem bestimmten charakteristischen Leseabstand zu verändern.

15. Vorrichtung (100; 200; 300; 400; 500; 600) nach einem der Ansprüche 1 bis 14, bei der das Empfangselement (120; 220; 320; 420; 520; 620) ein Empfangselement (122; 222; 322; 422; 522) umfasst, das im Inneren eines konischen Hohlraums (121; 221; 321; 421; 521) angeordnet ist, mit einem Winkel am Scheitel (A1; A2; A3; A4; A5) zwischen 15 und 45 Grad mit einer Tiefe (H1; H2; H3; H4; H5) von mindestens 30 Millimeter.

## Claims

1. A device (100, 200, 300, 400, 500, 600) for measuring a characteristic reading distance of an individual wearing an eyeglass frame (30) having ophthalmic lenses, the device comprising a measurement support (110, 210, 310, 410, 510, 610) carrying at least one ultrasound emitter member (120, 220, 320, 420, 520, 620) and at least one ultrasound receiver member (120, 220, 320, 420, 520, 620), at least one of these members, referred to as a main measurement member (120, 220, 320, 420, 520, 620), possessing a measurement axis (AM1, AM2, AM3, AM4, AM5) on which emission or reception by the main measurement member (120, 220, 320, 420, 520, 620) is privileged, and being suitable for emitting or receiving ultrasound propagating in a measurement cone (CM1, CM2, CM3, CM4, CM5) centered on its measurement axis (AM1, AM2, AM3, AM4, AM5), the device further including an electronic processor unit suitable for extracting from the ultrasound signal received by the receiver member (120, 220, 320, 420, 520, 620), that portion of said signal that is reflected by the lenses or the frame of eyeglass worn by the individual, to the exclusion of the portion of said signal that is reflected by the individual's skin, in order to deliver, as a function of the ultrasound signal received by the receiver member (120, 220, 320, 420, 520, 620), a signal that is representative of the distance (D) between the measurement support (110, 210, 310, 410, 510) and the eyeglass frame (30) worn by the individual's head (10), and to deduce therefrom said characteristic reading distance, and means (160, 360) to verify that said eyeglass frame is situated at least in part in the measurement cone of said main measurement member.

2. The device (100, 200, 300, 400) according to claim 1, including a reading medium presenting a plane display portion suitable for displaying signs and wherein the electronic processor unit is suitable for calculating said characteristic reading distance as being the distance between said reading medium (130, 530) and the eyeglass frame (30) worn by the individual's head (10).

3. The device (100, 200, 300, 400) according to claim 2, including means (140, 240, 340, 440) for releasably mounting the measurement support on the reading medium.

4. The device (100) according to claim 3, wherein the reading medium (130) is constituted by a computer screen and the releasable mounting means (140) are suitable for clipping the measurement support (110) onto the computer screen (130).

5. The device (500, 600) according to claim 2, including means (540) for releasably mounting the reading medium (530) on the measurement support (510, 610, 640).

6. The device (500, 600) according to claim 5, wherein said measurement support (510, 610, 640) forms a docking station for the reading medium (530), the docking station including both a base (511, 640) suitable for standing in stable manner on a plane, and releasable mechanical connection means (540) suitable for co-operating with complementary connection means of the reading medium.

7. The device according to any one of claims 1 to 6, wherein said main measurement member (120, 220, 320, 420) is mounted stationary on said measurement support (110, 210, 310, 410).

8. The device (500) according to any one of claims 1 to 6, wherein said main measurement member (520) is mounted to be movable on said measurement support (510) so as to be capable at least of pivoting relative to a pivot axis (X) perpendicular to the measurement axis (AM5) of said main measurement member (520).

9. The device (100, 200, 300, 400) according to any one of claims 1 to 8, wherein the means (160, 360) for verifying that the individual's head is situated at least in part in the measurement cone (CM1, CM2, CM3, CM4) of said main measurement member (120, 220, 320, 420, 520) include visual detection means (161, 162, 361, 362) operating on the parallax principle to enable the individual to detect visually a position of the individual's head relative to the main measurement member in which the head is situated at least in part in the measurement cone of said main measurement member.

10. The device (500) according to claim 8, wherein the means for verifying that the individual's head is situated at least in part in the measurement cone of said main measurement member include automatic detection means for automatically detecting a position of the individual's head (10) relative to the main measurement member (520), in which the head (10) is situated at least in part in the measurement cone (CM5) of said main measurement member (520), said automatic detection means including means for causing said main measurement member (520) to scan through a range of angular positions relative to the measurement support, and means for determining the angular position of said main measurement member (520) that corresponds to a received ultrasound signal of maximum intensity or intensity greater than a threshold.

11. The device (100, 200, 300, 400, 500, 600) according to claim 1, including a reading medium (130, 330, 430, 530) presenting a plane display portion (131, 331, 431, 531) suitable for displaying signs, and mechanical means for ensuring that the position of the reading medium (130, 330, 430, 530) relative to the measurement support is unchanging and in which the electronic processor unit is suitable for calculating said characteristic reading distance as a function of said unchanging position.

12. The device (100, 200, 300, 400) according to claim 1, including a reading medium (130, 330, 430) presenting a plane display portion (131, 331, 431) that is suitable for displaying signs, and means for determining the position of the reading medium relative to the measurement support in a measurement direction.

13. The device according to claim 12, the means for determining the relative position of the reading medium comprise means for measuring an angle of inclination of the measurement support relative to the horizontal and means for measuring the distance between the measurement support and the reading medium.

14. The device (100, 200, 300, 400, 500, 600) according to claim 1, including a reading medium (130, 330, 430, 530) presenting a plane display portion (131, 331, 431, 531) suitable for displaying signs, and an electronic processor unit suitable for controlling the reading medium to modify the size of the signs displayed as a function of the determined characteristic reading distance.

15. The device (100, 200, 300, 400, 500, 600) according to claim 1, wherein the receiver member (120, 220, 320, 420, 520, 620) includes a receiver element (122, 222, 322, 422, 522) placed inside a conical cavity (121, 221, 321, 421, 521) presenting an angle at the apex (A1, A2, A3, A4, A5) lying in the range 15 degrees to 45 degrees, and at a depth (H1, H2, H3, H4, H5) of at least 30 millimeters.
